# EUROPEAN PATENT APPLICATION

(11) **EP 4 141 884 A1**
(43) Date of publication of application: **01.03.2023**
(21) Application number: 21792328.3
(22) Date of filing: 22.04.2021
(51) Int. Cl.: G16H 50/20

(54) **PREDICTION SUPPORT SYSTEM, PREDICTION SUPPORT METHOD, PREDICTION SUPPORT PROGRAM, RECORDING MEDIUM, TRAINING DATASET, AND TRAINED MODEL GENERATING METHOD**

(30) Priority: 24.04.2020 JP 2020077135
(71) Applicant: OSAKA UNIVERSITY, Suita-shi Osaka 565-0871 (JP)
(72) Inventor: ASANOI, Hidetsugu, Suita-shi, Osaka 565-0871 (JP); SAWA, Yoshiki, Suita-shi, Osaka 565-0871 (JP); MIYAGAWA, Shigeru, Suita-shi, Osaka 565-0871 (JP); IKEGAWA, Sunao, Suita-shi, Osaka 565-0871 (JP)
(74) Representative: Grund, Martin
(86) International application number: PCT/JP2021/016284
(87) International publication number: WO 2021/215495

(57) **Abstract**

This invention relates to a prediction support system 1 for supporting prediction of the severity of a disease, comprising:
a detection device 2 for continuously detecting whether a patient with the disease is in bed,
an acquisition unit 33 for acquiring, based on the detection result of the detection device 2, an in-bed pattern D1 indicating, as a time series, whether the patient is in bed.

## Description

### Technical Field

The present invention relates to a technique of supporting prediction of the severity of a disease, and particularly relates to a technique of supporting the prediction based on a patient's in-bed pattern.

### Background Art

Management systems in which medical institutions use ICT to manage home care patients or home nursing patients have been commonly used (for example, Patent Literature 1). In the health management system described in Patent Literature 1, self-health management information, such as food and drink, physical activity, blood pressure level, heart rate, respiratory rate, body temperature, blood glucose level, weight, or the like of a patient is transmitted to a medical service providing device installed in a medical institution to manage the health conditions of the care recipient. If the patient's heartbeat, blood pressure, respiration, of the like are unstable, an alarm system notifies a patient's family or primary doctor so that medical assistance can be provided to the patient.

### Citation List

### Patent Literature

PTL 1: JP2009-223746A

### Summary of Invention

### Technical Problem

The number of heart failure patients has been increasing. In particular, it is predicted that elderly heart failure patients will reach 1.3 million by 2030. It has been known that once heart failure patients are admitted to a hospital with worsening symptoms, they do not return to their pre-worsening state even after leaving the hospital, and have a poor prognosis. It is therefore important to receive appropriate treatment as an outpatient before the condition becomes severe enough to require hospitalization.

However, even if the health management system of Patent Literature 1 is applied to a heart failure patient, it is not possible to identify signs of severe heart failure from physiological information, such as blood pressure level, heart rate, respiratory rate, and weight. Accordingly, in order to identify signs of severe heart failure, a patient needs to visit a medical institution to have at least a chest radiograph taken or a blood test performed, which imposes a heavy burden on the patient.

The present invention was made to solve the above problems, and aims to support prediction of the severity of a disease without imposing a burden on the patient.

### Solution to Problem

In order to solve the above problems, the present invention includes the following aspects.

### Item 1.

A prediction support system for supporting prediction of the severity of a disease, comprising
a detection device for continuously detecting whether a patient with the disease is in bed,
an acquisition unit for acquiring, based on the detection result of the detection device, an in-bed pattern indicating, as a time series, whether the patient is in bed.

### Item 2.

The prediction support system according to Item 1, further comprising a prediction unit for predicting the severity of the disease based on the in-bed pattern.

### Item 3.

The prediction support system according to Item 2, wherein the prediction unit predicts the severity of the disease using a trained model in which the correlation between the in-bed pattern of the patient with the disease and the severity of the disease is machine-trained.

### Item 4

The prediction support system according to Item 2 or 3, wherein the prediction unit predicts the severity as a probability that the patient will require hospitalization within a predetermined period of time.

### Item 5

The prediction support system according to any one of Items 1 to 4, wherein the disease is heart failure, pneumonia, or dementia.

### Item 6

The prediction support system according to Item 5, wherein the disease is heart failure.

### Item 7

A prediction support program for operating a computer as a unit of the prediction support system according to any one of Items 1 to 6.

### Item 8

A computer-readable recording medium in which the prediction support program according to Item 7 is saved.

### Item 9

A prediction support method for supporting prediction of the severity of a disease, comprising
a detection step of continuously detecting whether a patient with the disease is in bed, and
an acquisition step of acquiring, based on the detection result of the detection step, an in-bed pattern indicating, as a time series, whether the patient is in bed.

### Item 10

A method for generating a dataset for training, comprising
a detection step of continuously detecting whether a patient with a disease is in bed,
an acquisition step of acquiring, based on the detection result of the detection step, an in-bed pattern indicating, as a time series, whether the patient is in bed, and
a training dataset generation step of generating a dataset for training by correlating the in-bed pattern with the severity of the patient's disease.

### Item 11

A method for generating a trained model, comprising performing machine training using the dataset for training generated by the method according to Item 10, thereby generating a trained model in which the in-bed pattern of an unknown patient with the disease is an input, and the severity of the disease of the unknown patient is an output.

### Advantageous Effects of Invention

The present invention can support prediction of the severity of a disease without imposing a burden on the patient.

### Brief Description of Drawings

Fig. 1 is a block diagram showing a schematic structure of a prediction support system according to one embodiment of the present invention.
Fig. 2 is a schematic diagram showing an example of the installation of the prediction support system.
Fig. 3 shows an example of the in-bed pattern of a patient with moderate heart failure.
Fig. 4 shows an example of the in-bed pattern of a patient with severe heart failure.
Fig. 5 is a flowchart showing a procedure of a prediction support method according to one embodiment of the present invention.
Fig. 6 is a flowchart showing a procedure of a method for generating a trained model.

### Description of Embodiments

The embodiments of the present invention are explained below with reference to the attached drawings. The present invention is not limited to the following embodiments.

### System Structure

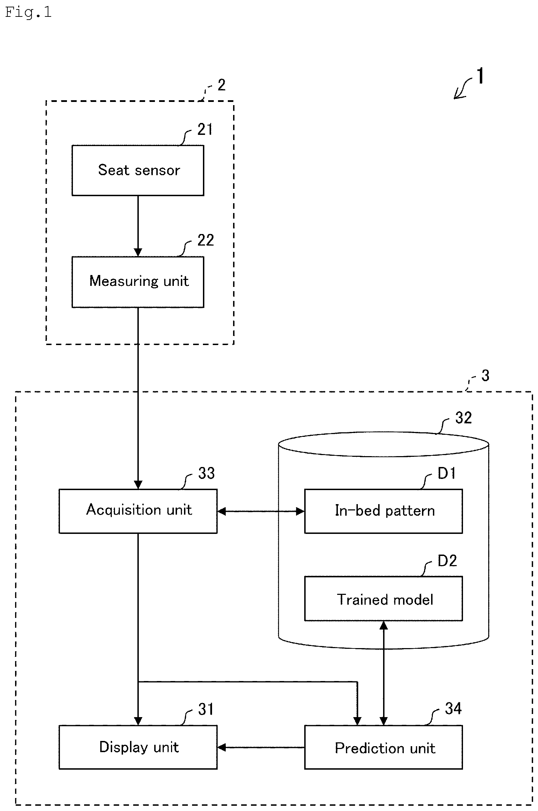

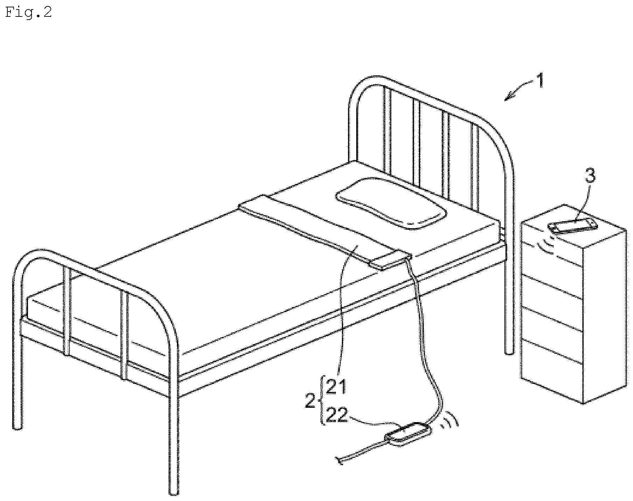

Fig. 1 is a block diagram showing a schematic structure of a prediction support system 1 according to one embodiment of the present invention. Fig. 2 is a schematic diagram showing an example of the installation of the prediction support system 1. The prediction support system 1 is a system for supporting the prediction of the severity of a disease, which comprises a detection device 2 and a management device 3. The prediction support system 1 is provided in a place where a patient with a disease resides (e.g., at home or in a nursing home). The main disease targeted by the present invention is heart failure, pneumonia, or dementia; in this embodiment, a patient with heart failure is a target.

The detection device 2 is a device for continuously detecting whether a patient is in bed. The detection device 2 comprises a seat sensor 21 and a measuring unit 22.

As shown in Fig. 2, the seat sensor 21 is a piezoelectric seat sensor formed of a thin, soft strip piezoelectric rubber, and is placed on the bed that the patient routinely uses. In Fig. 2, a sheet and a quilt that are placed on the seat sensor 21 are omitted. While the patient is in bed, pressure is applied to the seat sensor 21, and the seat sensor 21 generates an electrical signal due to the piezoelectric effect.

In this embodiment, being in bed means that a patient is lying down regardless of whether the patient is sleeping. Accordingly, the seat sensor 21 may be provided not only in a bed but also at other places (e.g., a sofa) where the patient can lie down to rest.

The measuring unit 22 is connected to the seat sensor 21, and converts the electrical signal generated by the seat sensor 21 into a digital signal (in-bed occupancy signal). The measuring unit 22 has the function of communicating with the management device 3 by using Bluetooth (registered trademark), and transmits the digital signal to the management device 3 periodically (e.g., every second) as the detection result of the detection device 2.

As shown in Fig. 2, the management device 3 includes a smartphone. The management device 3 may consist of a general-purpose computer, or it may be located in the cloud, as long as it can communicate directly or indirectly with the measuring unit 22. The function of the management device 3 may be built in the detection device 2, and the detection device 2 and the management device 3 can be configured as a single device.

As shown in Fig. 1, the management device 3 comprises a display unit 31, a storage unit 32, an acquisition unit 33, and a prediction unit 34.

The display unit 31 can be formed of, for example, a liquid crystal display or an organic EL display. The storage unit 32 can be formed of, for example, a flash memory, and stores various data comprising in-bed pattern D1 and trained model D2.

Each of the acquisition unit 33 and the prediction unit 34 may be achieved by a logic circuit or the like based on hardware, or by a CPU or the like based on software. When each of these units is achieved based on the software, the CPU of the management device 3 reads the prediction support program of the present invention in the main storage device to execute the program, thus achieving the unit. The prediction support program may be downloaded to the management device 3 via a communication network, such as the internet, or the prediction support program may be saved on a computer-readable, non-transitory storage medium, such as an SD card, and installed in the management device 3 via the storage medium.

The acquisition unit 33 has the function of acquiring, based on the detection result of the detection device 2, the in-bed pattern D1 indicating, as a time series, whether the patient is in bed. Specifically, the acquisition unit 33 receives a digital signal indicating whether the patient is in bed from the measuring unit 22, and stores the signal in the storage unit 32. Thereby, detection of whether the patient is in bed as a time series is accumulated as the in-bed pattern D1.

The relationship between the in-bed pattern D1 and the severity (degree of progression) of the disease is explained below.

It has been known that in various diseases, as the patient's condition worsens or progresses, the patient lies in bed not only at night but also during the day, thus increasing the time in bed. For example, with regard to patients with heart failure, clinical studies conducted by the inventors of the present application showed a tendency for the time in bed (lying time) to increase before the heart failure worsened. However, it was impossible to predict pathology from the total bed time because the time in bed varied greatly from person to person, depending on the life cycle and other factors.

On the other hand, aging or onset and exacerbation of disease lead to changes in a people's behavior pattern. Specifically, in the case of a patient with heart failure, as the condition progresses, water is stored in the body (fluid retention syndrome). When the patient leaves bed, water is stored in the legs; however, when the patient is in bed, the water level is the same between the legs and the heart. This lowers the blood pressure in the leg veins; accordingly, water returns to the vessels, increasing the amount of blood returning to the heart to increase the heart load, which makes the condition of heart failure more likely to proceed. In addition, blood flow to the kidneys increases, which causes night urination. Further, as the condition of heart failure progresses, the sympathetic nervous system becomes constantly tense, which consequently causes symptoms such as increased tossing and turning during sleep and arousal due to lack of deep sleep, resulting in sleep fragmentation. As the condition further progresses, the pattern of life changes to one in which the patient is in bed during the day.

The inventors of the present application have focused on the fact that such changes in the behavior pattern due to poor health are keenly reflected in the daily in-bed pattern, and have found that analyzing the in-bed pattern over time, rather than simply analyzing the time in bed, can result in understanding signs of disease exacerbation.

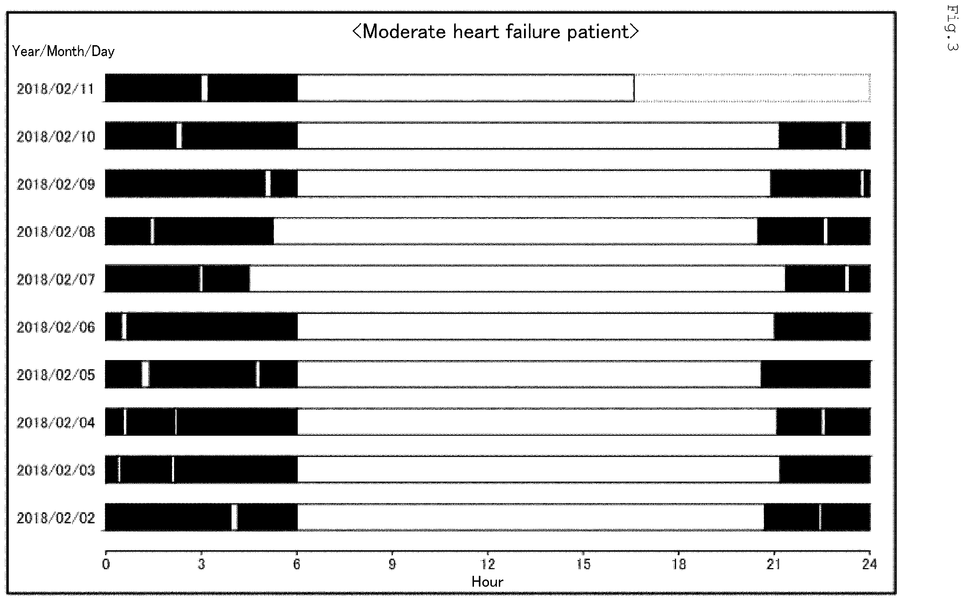

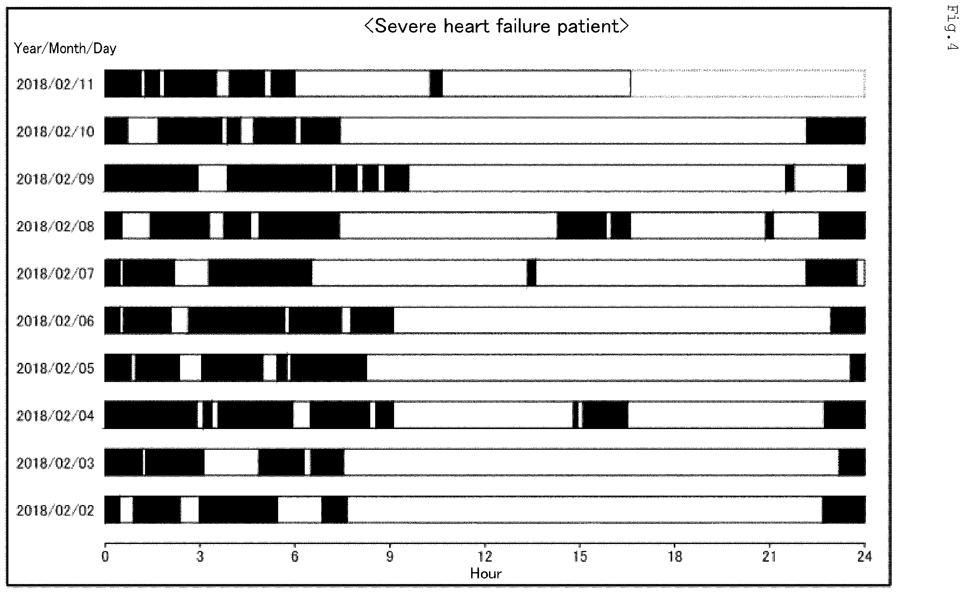

Fig. 3 shows an example of an in-bed pattern of a patient with moderate heart failure, and Fig. 4 shows an example of an in-bed pattern of a patient with severe heart failure. In Figs. 3 and 4, the timeline of one day, which extends horizontally, is arranged vertically in a matrix; the timeline when the patient is in bed is shown in black, and the timeline when the patient is out of bed is shown in white.

The in-bed pattern shown in Fig. 3 indicates that the patient is out of bed once or twice during the night, and not in bed during the day. In contrast, the in-bed pattern shown in Fig. 4 indicates that as the patient leaves bed more frequently and for a longer time during the night, the patient is in bed during the day on some days. Thus, as the heart failure proceeds, the in-bed pattern regularity is disturbed.

The prediction unit 34 has the function of predicting the severity of the disease based on in-bed pattern D1. In this embodiment, the prediction unit 34 predicts the severity of heart failure by inputting the in-bed pattern D1 of a predetermined period (e.g., 30 days) into the trained model D2 in which a correlation between the in-bed pattern of a heart failure patient and the severity of heart failure is machine-trained. The method for generating trained model D2 is described below. The manner in which the severity is expressed is not particularly limited; in this embodiment, the prediction unit 34 predicts the probability that the patient will be in a condition requiring hospitalization within a predetermined period (e.g., within 30 days) as the severity. The prediction result of the prediction unit 34 is displayed on the display unit 31, and transmitted via the internet to a predetermined medical institution.

### Procedure

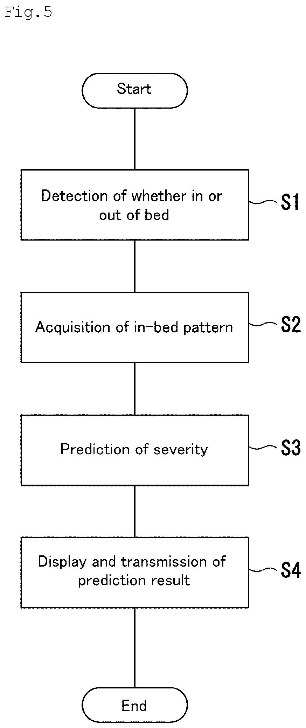

Fig. 5 is a flowchart showing the procedure of the prediction support method for supporting the prediction of the severity of the disease using the prediction support system 1 according to one embodiment of the present invention. In step S1 (detection step), the detection device 2 continuously detects whether the patient is in bed. In step S2 (acquisition step), based on the detection result of step S1, the acquisition unit 33 acquires an in-bed pattern D1 that indicates, as a time series, whether the patient is in bed. In step S3 (prediction step), the prediction unit 34 predicts the severity of the disease based on the in-bed pattern D1. In step S4, the prediction result is displayed on the display unit 31 and also transmitted to a medical institution or the like.

As described above, in this embodiment, the severity of the disease is predicted based on the in-bed pattern D1, which indicates, as a time series, whether the patient is in bed. This enables the patient and healthcare professionals, such as a primary doctor, to constantly understand the patient's current condition and to predict possible future situations. In addition, although the prognosis for heart failure patients particularly becomes worse once the condition becomes severe enough to require hospitalization, the embodiment of the present invention enables early therapeutic intervention by using the in-bed pattern to detect exacerbation of heart failure at an early stage. As a result, disease exacerbation or hospital admission can be reduced, leading not only to a better quality of life for patients but also to reduced healthcare costs.

Since the in-bed pattern D1 can be collected using the detection device 2, which is formed of the seat sensor 21, there is no need to restrain the patient and attach the sensor, or the patient does not need to visit a medical institution. Accordingly, the prediction of the severity of a disease can be supported without imposing a burden on the patient. Additionally, since the in-bed pattern D1 is data showing whether the patient is in bed, the load on communication equipment is low, and construction of the prediction support system 1 is easy.

### Method for Generating Trained Model

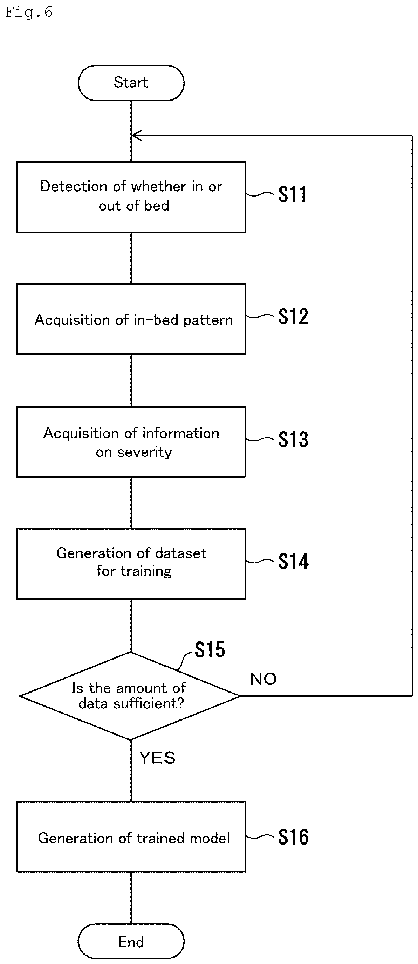

Subsequently, the trained model D2 is explained. Fig. 6 is a flowchart showing the procedure of the method for generating the trained model D2. First, whether a heart failure patient in home care is in bed is continuously detected using the detection device 2 shown in Figs. 1 and 2 (step S11, detection step), and the patient's in-bed pattern is acquired based on the detection result of step S1 (step S12, acquisition step).

In step S13, information on the severity of the heart failure patient is acquired. In this embodiment, information on severity is the experience of hospitalization and the length of time before hospitalization. For example, if the patient is admitted to the hospital within a predetermined period (within 30 days) after acquiring the in-bed pattern, the number of days from the last day of the acquisition of the in-bed pattern to the hospitalization is used as information on severity.

In step S4 (training dataset generation step), a training dataset (teacher data) is generated by associating the in-bed pattern with the patient's disease severity. Several training datasets can be generated from a single patient. For example, if the 60-day in-bed pattern is acquired from the patient, thirty 30-day in-bed patterns (with day 1 defined as the starting point to day 30) are extracted from the 60-day in-bed pattern. By associating the number of days between the last day of the in-bed pattern and the date of admission to the hospital with each of the extracted in-bed patterns, 30 items of training data can be generated.

By repeating the processing of steps S11 to S14, the training data are accumulated, and a dataset for training is generated. The processing of steps S11 to S14 is performed on several patients until the amount of data in the dataset for training is sufficient (YES in step S15).

Thereafter, in step S16 (trained model generation step), by performing machine training using a dataset for training, a trained model D2 in which a bed pattern of an unknown patient with a disease is an input, and the severity of the disease of the unknown patient is an output, is generated. The machine-training method is not particularly limited; deep learning can be used.

### Additional Information

Although the embodiment of the present invention is described, the present invention is not limited to the above embodiment, and various changes are possible as long as they do not depart from the intent of the invention.

In the above embodiment, the prediction unit 34 uses the trained model D2 to predict the severity of the disease; however, the prediction may be performed using, for example, an image analysis technique, without using an artificial intelligence algorithm. Furthermore, it is also possible that the in-bed pattern D1 for a predetermined period may be displayed on the display unit 31, or is transmitted to a medical institution or the like, so that a doctor or the like can determine the severity of the disease by visually examining the in-bed pattern D1, without using the prediction unit 34.

In the above embodiment, the detection device having a piezoelectric seat sensor is used to detect whether the patient is in bed; however, the means of detecting whether the patient is in bed is not particularly limited. For example, whether the patient is in bed can be determined by using a camera or a motion sensor. Even by this means, the patient does not have to visit a medical institution, and is not forced to bear a burden.

As the detection device 2 shown in Figs. 1 and 2, it is preferable to use a body motion sensor produced by Sumitomo Science & Engineering Co., Ltd. The body motion sensor can simultaneously measure biometric information (vital data) such as heartbeat and respiration. This enables clearly distinguishing the case in which an object is placed on the bed from the case in which a person is in bed, which makes it possible to more accurately detect whether the patient is in bed.

Although heart failure is taken as a target of the prediction of severity in the above embodiment, there is no limitation as long as the disease is such that the in-bed pattern of the patient is changed according to the severity. Examples of the disease include pneumonia and dementia.

In the case of pneumonia patients, as the disease progresses, inflammation of the pulmonary interstitium occurs, which causes a sensor in the pulmonary interstitium, which controls breathing, to fail to properly work, resulting in disrupted breathing, which prevents deep sleep and makes it easier to wake up. This results in fragmented night-time sleep and an altered in-bed pattern.

In the case of dementia patients, e.g., those with Alzheimer's disease, neurodegeneration of cholinergic neurons in the basal ganglia of the myelinated nucleus, nucleus accumbens, and nucleus accumbens, and noradrenergic neurons in the brainstem, etc., leads to a decrease in REM sleep, leading to REM sleep behavior disorders and sleep-disordered breathing, resulting in fragmented night-time sleep and an altered in-bed pattern.

### Examples

Examples of the present invention are described below. The present invention is not limited to the following examples.

The inventors of the present application conducted a clinical study from August 2017 to March 2019, using a piezoelectric seat sensor to collect in-bed signals every second, which indicate whether a patient is in bed. Specifically, in-bed signals of 18 heart failure patients were collected every day. During the period of the clinical study, 14 hospitalization events due to heart failure exacerbation occurred. It was observed that regularity was disturbed in the 30-day in-bed pattern of a hospitalized patient before hospitalization (Fig. 4), as compared to the in-bed pattern of a patient whose condition was stable without hospitalization (Fig. 3), and that these patterns were significantly different.

This difference indicates the following. By conducting machine training using a dataset for training in which the experience of hospitalization and the period until hospitalization are associated with each of the in-bed patterns acquired from heart failure patients to thereby generate a trained model, the severity of unspecified heart failure patients can be predicted based on the in-bed pattern using the trained model. In particular, it is expected that the present invention is effective for detecting pathological changes in heart failure patients who are repeatedly readmitted to hospital in an early stage.

### Explanation of Description

1 Prediction support system
2 Detection device
21 Seat sensor
22 Measuring unit
3 Management device
31 Display unit
32 Storage unit
33 Acquisition unit
34 Prediction unit
D1 In-bed pattern
D2 Trained model

## Claims

1. A prediction support system for supporting prediction of the severity of a disease, comprising:
a detection device for continuously detecting whether a patient with the disease is in bed,
an acquisition unit for acquiring, based on the detection result of the detection device, an in-bed pattern indicating, as a time series, whether the patient is in bed.

2. The prediction support system according to claim 1, further comprising a prediction unit for predicting the severity of the disease based on the in-bed pattern.

3. The prediction support system according to claim 2, wherein the prediction unit predicts the severity of the disease using a trained model in which the correlation between the in-bed pattern of the patient with the disease and the severity of the disease is machine-trained.

4. The prediction support system according to claim 2 or 3, wherein the prediction unit predicts the severity as a probability that the patient will require hospitalization within a predetermined period of time.

5. The prediction support system according to any one of claims 1 to 4, wherein the disease is heart failure, pneumonia, or dementia.

6. The prediction support system according to claim 5, wherein the disease is heart failure.

7. A prediction support program for operating a computer as a unit of the prediction support system according to any one of claims 1 to 6.

8. A computer-readable recording medium in which the prediction support program according to claim 7 is saved.

9. A prediction support method for supporting prediction of the severity of a disease, comprising
a detection step of continuously detecting whether a patient with the disease is in bed, and
an acquisition step of acquiring, based on the detection result of the detection step, an in-bed pattern indicating, as a time series, whether the patient is in bed.

10. A method for generating a dataset for training, comprising
a detection step of continuously detecting whether a patient with a disease is in bed,
an acquisition step of acquiring, based on the detection result of the detection step, an in-bed pattern indicating, as a time series, whether the patient is in bed, and
a training dataset generation step of generating a dataset for training by correlating the in-bed pattern with the severity of the patient's disease.

11. A method for generating a trained model comprising performing machine training using the dataset for training generated by the method according to claim 10, thereby generating a trained model in which the in-bed pattern of an unknown patient with the disease is an input, and the severity of the disease of the unknown patient is an output.
